Europäisches Patentamt

European Patent Office

Office européen des brevets

(19)

(11) Publication number: **0 640 333 A2**

## (12) EUROPEAN PATENT APPLICATION

(21) Application number: **94112356.4**

(22) Date of filing: **08.08.94**

(51) Int. Cl.6: **A61K 7/06**

(30) Priority: **09.08.93 JP 217981/93**

(43) Date of publication of application:
**01.03.95 Bulletin 95/09**

(84) Designated Contracting States:
**AT BE CH DE DK ES FR GB GR IE IT LI LU MC NL PT SE**

(71) Applicant: **TSUMURA & CO.**
**4-10, 3-chome, Nihonbashi**
**Chuo-ku,**
**Tokyo-to (JP)**

(72) Inventor: **Yasuda, Minoru, c/o Tsumura & Co.**
**12-7, 2-bancho,**
**Chiyoda-ku**
**Tokyo (JP)**
Inventor: **Tsunakawa, Mitsuo, c/o Tsumura & Co.**
**Shizuoka Factory,**
**392, Tsukiji**
**Fujieda-shi,**
**Shizuoka-ken (JP)**

Inventor: **Matsuo, Koji, c/o Tsumura & Co.**
**12-7, 2-bancho,**
**Chiyoda-ku**
**Tokyo (JP)**
Inventor: **Aoshima, Katsunori, c/o Tsumura & Co.**
**Shizuoka Factory,**
**392, Tsukiji**
**Fujieda-shi,**
**Shizuoka-ken (JP)**
Inventor: **Ohta, Masahiro**
**2-23-14, Momoi,**
**Suginami-ku**
**Tokyo (JP)**
Inventor: **Kinno, Shigehiro**
**3-12-35, Nagano**
**Gyoda-shi,**
**Saitama-ken (JP)**

(74) Representative: **Barz, Peter, Dr.**
**Schmied-Kowarzik & Partner**
**Siegfriedstrasse 8**
**D-80803 München (DE)**

(54) **Hair growth promoting aerosol formulation.**

(57) A hair growth promoting aerosol formulation having a hair growth promoter composition in a gel form containing as effective components a crude drug extract having an effect of promoting the circulation of blood and a vitamin or a derivative thereof, and a water-soluble polymer by which the hair growth promoter composition is gelatinized; and a propellant. There is also disclosed an aerosol product of a hair growth promoter prepared by filling the above-mentioned hair growth promoting aerosol formulation in an aerosol container provided with a nozzle having a diameter of 1 mm or less.

EP 0 640 333 A2

The present invention relates to a hair growth promoting aerosol formulation having not only hair growth promoting effects, but also appropriate hair setting effects. In addition, the present invention also relates to an aerosol product using the above-mentioned hair growth promoting aerosol formulation.

Conventional hair growth promoters are in a liquid form to be directly applied onto the scalp. This kind of hair growth promoters can impart a cooling sensation to the users, but cannot contribute to the setting of their hair.

In recent years, many aerosol products of hair growth promoters have entered the market. These hair growth promoters are also in liquid form. This causes a problem where the hair growth promoter tends to drip from the end of the hairs immediately after spraying onto the scalp. In addition to this, these hair growth promoters do not provide the hair setting effect.

There is an increasing demand for the development of a hair growth promoting aerosol formulation having not only the hair growth promoting effect, but also the hair setting effect, and an aerosol product using such a hair growth promoting aerosol formulation.

There is proposed an aerosol formulation for the human body as disclosed in Japanese Laid-Open Patent Application 4-89423, which is characterized by comprising a propellant and a composition in a gel form which is obtained by use of a carboxyvinyl polymer, that is a water-soluble polymer serving to gelatinize the composition as used in the present invention.

In this application there is a technical idea of applying the formulation in the gel form to an aerosol product, but not to an aerosol product of a hair growth promoter. Specifically, no suggestion is made in the application of the effective components of a hair growth promoter, that is, a hair growth promoter capable of smoothly reaching the scalp and setting the hairs to a proper extent.

Furthermore, in the above-mentioned application, there is no technical idea of preparing an aerosol product using the formulation which contains as the effective component the extract of a crude drug having an effect of promoting the circulation of the blood. Conventionally, in the case where the aerosol formulation is made using the extract of a crude drug, sediments are generated, and the settling sediments are accumulated at the bottom of an aerosol container during the storage. For the spraying operation, the contents are pumped from the bottom of the container at one stroke, so that a dipping tube, a valve member, and a nozzle are easily clogged with the accumulated sediments.

Accordingly, a first object of the present invention is to provide a hair growth promoting aerosol formulation providing both the hair growth promoting effect and the hair setting effect, in addition, imparting a cooling sensation to the scalp without dripping immediately after application, and which will not clog the valve and other members when filled in the aerosol container.

A second object of the present invention is to provide an aerosol product using the above-mentioned hair growth promoting aerosol formulation.

The above-mentioned first object of the present invention can be achieved by a hair growth promoting aerosol formulation comprising a hair growth promoter composition in the form of a gel, comprising as effective components a crude drug extract having an effect of promoting the circulation of blood and a vitamin or a derivative thereof, and a water-soluble polymer by which the hair growth promoter composition is gelatinized, and a propellant.

The second object of the present invention can be achieved by an aerosol product of a hair growth promoter prepared by filling a hair growth promoting aerosol formulation comprising a hair growth promoter composition in the form of a gel, comprising as effective components a crude drug extract having an effect of promoting the circulation of blood and a vitamin or a derivative thereof, and a water-soluble polymer by which the hair growth promoter composition is gelatinized, and a propellant in an aerosol container provided with a nozzle having a diameter of 1 mm or less.

A more complete appreciation of the invention and many of the attendant advantages thereof will be readily obtained as the same becomes better understood by reference to the following detailed description when considered in connection with the accompanying drawing, wherein:

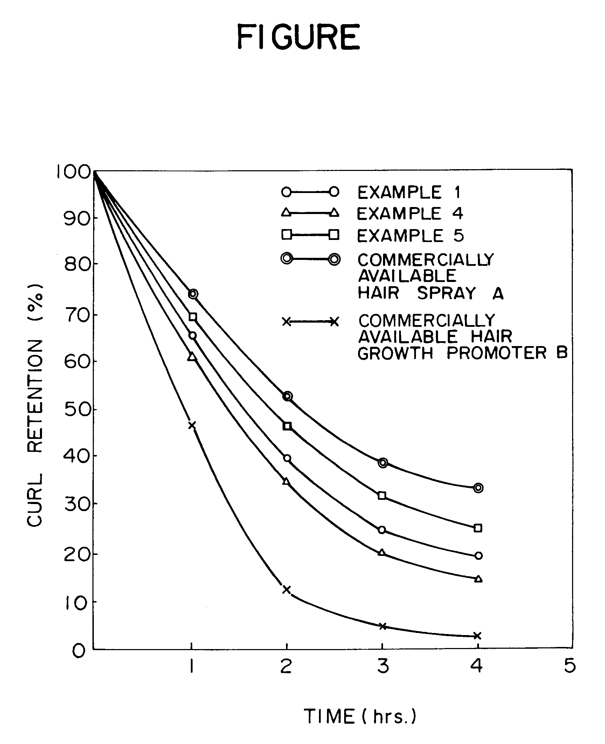

Fig. 1 is a graph explaining the hair setting effect of the hair growth promoters according to the present invention, in terms of the curl retention ratio of the hair.

The hair growth promoting aerosol formulation according to the present invention comprises as effective components (a) a crude drug extract having the effect of promoting the circulation of blood and (b) a vitamin or a derivative thereof.

Specific examples of the crude drug extract for use in the present invention include Japanese Angelica Root (Angelicae Radix), Cnidium Rhizome (Cnidii Rhizoma), Panax Rhizome (Panacis Japonici Rhizoma), Ginseng Root (Ginseng Radix), Swertia Herb (Swertiae Herba), Ginger Rhizome (Zingiberis Rhizoma), Rehmannia Root (Rehmanniae Radix), Aloe (Aloe), Glycyrrhiza Root (Glycyrrhizae Radix), Garlic, Capsicum (Capsici Fructus), and the like.

The above-mentioned crude drug may be extracted with water, a lower alcohol such as methanol or ethanol, or a polyhydric alcohol such as propylene glycol or 1,3-butylene glycol in an amount of 10 to 20 times the amount of the crude drug to be employed at room temperature or a temperature lower than the boiling temperature of the solvent employed.

The extract thus obtained may be concentrated and dried for use in the hair growth promoting aerosol formulation of the present invention.

The extract of the aforementioned crude drug may be used alone or in combination.

It is preferable that the amount of the crude drug extract be in the range from 1.5 to 30 wt.% of the total weight of the hair growth promoter composition to obtain sufficient hair growth promoting effect. This will also maintain the viscosity of the hair growth promoter composition and assist in assessing manufacturing costs.

The vitamin or a derivative thereof contained in the hair growth promoter composition can activate the metabolism which will facilitate the restoration of the damaged cuticles of hairs.

Examples of the vitamin are tocopherol, vitamin A, vitamin B, vitamin C, vitamin D, vitamin F, vitamin H, vitamin K, vitamin P, vitamin U, D-panthenol (pantothenyl alcohol), carnitine, ferulic acid, $\gamma$-oryzanol, lipoic acid, and orotic acid, and derivatives thereof.

It is preferable that the amount of the vitamin be in the range of 0.5 to 10 wt.% of the total weight of the hair growth promoter composition to obtain the previously mentioned effect of the vitamin.

A water-soluble polymer for use in the hair growth promoter composition serves to thicken the aqueous medium comprising the aforementioned crude drug extract and vitamin, and gelatinize the same. In addition, the water-soluble polymer can contribute to the hair setting effect.

Examples of the water-soluble polymer for use in the present invention include natural polymers such as carrageenan, furcellaran, locust-bean gum and xanthane gum; semisynthetic polymers such as hydroxymethyl cellulose, hydroxyethyl cellulose, hydroxypropyl cellulose, hydroxypropylmethyl cellulose, carboxymethyl cellulose and ethyl cellulose; and synthetic polymers such as carboxyvinyl polymer, polyvinyl pyrrolidone, and a copolymer of vinyl pyrrolidone and vinyl acetate. These water-soluble polymers can be used alone or in combination.

The aforementioned carboxyvinyl polymer serving as the water-soluble polymer for use in the present invention is a hydrophilic crosslinked polymer with acrylic acid as the main component. Commercially available products, such as "Hivis Wako 103", "Hivis Wako 104" and "Hivis Wako 105" (Trademark), made by Wako Pure Chemical Industries, Ltd., and "Carbopol 934", "Carbopol 940" and "Carbopol 941" (Trademark), made by B,F, Goodrich Chemical Company can be employed.

In order to prevent the hair growth promoter from dripping off the scalp and the hair after application, and to obtain the desired hair setting effect, the water-soluble polymer is added to the hair growth promoter composition to such a degree that the viscosity of the hair growth promoter composition is maintained within the range of 5,000 to 100,000 cp, preferably in the range of 5,000 to 30,000, more preferably 8,500 to 30,000 cp.

The viscosity of the hair growth promoter composition as specified above can be measured in accordance with the Japanese Standards of Cosmetic Ingredients, Second Edition, released by Yakuji Nippo, Ltd. More specifically, using the Brookfield viscometer, the viscosity of the hair growth promoter composition is measured by rotating No. 4 rotor at the speed of 12 rpm at 20°C.

The hair growth promoter composition may further comprise a perfume, purified water, a lower alcohol, a refrigerant, a preservative, a humectant, a chelating agent, and other effective components and auxiliary components which are conventionally employed.

For example, the chelating agent can effectively prevent the generation of sediments caused by the addition of the crude drug extract. Examples of the chelating agent are ethylenediaminetetraacetic acid - 2Na (EDTA-2Na), ethylenediaminetetraacetic acid - 3Na (EDTA-3Na), ethylenediaminetetraacetic acid - 4Na (EDTA-4Na), phosphoric acid, citric acid and succinic acid. It is preferable that the amount of the chelating agent be in the range of 0.01 to 0.10 wt.%, more preferably in the range of 0.02 to 0.05 wt.% of the total weight of the hair growth promoter composition.

The hair growth promoting aerosol formulation according to the present invention comprises the previously mentioned hair growth promoter composition in a gel form, and a propellant.

Although the choice of propellant is not restricted in this invention, the use of dimethyl ether is preferred. In addition, liquefied natural gas, carbon dioxide gas or chlorofluorocarbon may be used in combination with dimethyl ether for the propellant.

The desired weight ratio of the hair growth promoter composition to the propellant is in the range of 9:1 to 1:9. This will vary depending upon the formulations and the kinds of hair growth promoter composition and propellant.

According to the present invention, a mixture of the hair growth promoter composition and the propellant is filled into an aerosol container provided with a nozzle having a diameter of 1 mm or less, preferably in the range of 0.4 to 0.8 mm. Jet-spraying can be attained by use of such an aerosol container, so that the effective components contained in the hair growth promoter in a gel form can reach the scalp. Thus, the excellent hair setting effect can be obtained, and the hair growth promoting effect can be increased. In this case, the internal pressure of the aerosol container may be controlled to 2 to 7 $kg/cm^2$ at 25°C.

According to the present invention, the hair growth promoter composition comprises the extract of the crude drug and the vitamin which affect and promote the hair growth, with the water-soluble polymer serving as the gelatinizer. The thus obtained hair growth promoter composition in a gel form used in combination with the propellant not only promotes excellent hair growth but also provides proper hair setting effects.

The hair growth promoting aerosol formulation according to the present invention can be sprayed onto the scalp in a good condition without dripping from the scalp and the hairs because the hair growth promoter composition is in the form of a gel and has an appropriate viscosity.

Furthermore, even though sediments may be generated from the crude drug extract, the sediments will remain in the original position, and will not settle and accumulate at the bottom of the aerosol container because of the relatively large viscosity of the hair growth promoter composition. As a result, the valve and other members in the aerosol container are not clogged with the sediments accumulated at the bottom. The amount of sediments locally generated in the aerosol container is so slight that the quality of the hair growth promoter is not affected when used on the hair.

In addition, according to the present invention, the mixture of the hair growth promoter composition and the propellant is filled into an aerosol container having a nozzle with a diameter of 1 mm or less. Therefore, the hair growth promoter can reach the scalp directly, so that the hair setting effect and hair growth promoting effect are further enhanced.

Other features of this invention will become apparent in the course of the following description of exemplary embodiments, which are given for illustration of the invention and are not intended to be limiting thereof.

**Example 1**

The following components were mixed to prepare a hair growth promoter composition:

|  | Wt.% |
|---|---|
| D-panthenol | 0.50 |
| Extract of Swertia Herb (Swertiae Herba) | 1.00 |
| Extract of Ginseng Root (Ginseng Radix) | 0.50 |
| Ethanol | 21.00 |
| EDTA-2Na | 0.03 |
| Diisopropanolamine (neutralizing agent) | 0.29 |
| 2% aqueous solution of carboxyvinyl polymer | 14.50 |
| Perfume (including 1-menthol) | 0.20 |
| Preservative | 0.10 |
| Ion-exchange water (purified water) | balance |

66.7 parts by weight of the above prepared hair growth promoter composition with a viscosity of 22,700 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 $kg/cm^2$ at 25°C.

**Example 2**

The following components were mixed to prepare a hair growth promoter composition:

|  | Wt.% |
|---|---|
| Tocopherol | 0.50 |
| Extract of Glycyrrhiza Root (Glycyrrhizae Radix) | 1.00 |
| Extract of Swertia Herb (Swertiae Herba) | 0.50 |
| Ethanol | 21.00 |
| EDTA-2Na | 0.03 |
| Diisopropanolamine (neutralizing agent) | 0.23 |
| 2% aqueous solution of carboxyvinyl polymer | 11.50 |
| Perfume (including 1-menthol) | 0.20 |
| Preservative | 0.10 |
| Ion-exchange water (purified water) | balance |

66.7 parts by weight of the above prepared hair growth promoter composition with a viscosity of 9,800 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25 °C.

**Example 3**

The following components were mixed to prepare a hair growth promoter composition:

|  | Wt.% |
|---|---|
| Tocopheryl acetate | 0.50 |
| Extract of Swertia Herb (Swertiae Herba) | 1.00 |
| Extract of Garlic | 0.50 |
| Ethanol | 21.00 |
| EDTA-2Na | 0.03 |
| Diisopropanolamine (neutralizing agent) | 0.24 |
| 2% aqueous solution of carboxyvinyl polymer | 12.00 |
| Perfume (including 1-menthol) | 0.20 |
| Preservative | 0.10 |
| Ion-exchange water (purified water) | balance |

66.7 parts by weight of the above prepared hair growth promoter composition with a viscosity of 11,100 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25 °C.

**Example 4**

The following components were mixed to prepare a hair growth promoter composition:

|  | Wt.% |
|---|---|
| D-panthenol | 0.50 |
| Extract of Swertia Herb (Swertiae Herba) | 1.00 |
| Extract of Ginseng Root (Ginseng Radix) | 0.50 |
| Ethanol | 21.00 |
| EDTA-2Na | 0.03 |
| Diisopropanolamine (neutralizing agent) | 0.25 |
| 2% aqueous solution of carboxyvinyl polymer | 12.50 |
| Perfume (including 1-menthol) | 0.20 |
| Preservative | 0.10 |
| Ion-exchange water (purified water) | balance |

66.7 parts by weight of the above prepared hair growth promoter composition with a viscosity of 12,450 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25 °C.

**Example 5**

The following components were mixed to prepare a hair growth promoter composition:

|  | Wt.% |
|---|---|
| D-panthenol | 0.50 |
| Extract of Swertia Herb (Swertiae Herba) | 1.00 |
| Extract of Ginseng Root (Ginseng Radix) | 0.50 |
| Ethanol | 30.00 |
| EDTA-2Na | 0.03 |
| Diisopropanolamine (neutralizing agent) | 0.29 |
| 2% aqueous solution of carboxyvinyl polymer | 14.50 |
| Polyvinyl pyrrolidone derivative | 1.00 |
| Perfume (including 1-menthol) | 0.20 |
| Preservative | 0.10 |
| Ion-exchange water (purified water) | balance |

66.7 parts by weight of the above prepared hair growth promoter composition with a viscosity of 17,300 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25 °C.

**Example 6**

The following components were mixed to prepare a hair growth promoter composition:

|  | Wt.% |
| --- | --- |
| D-panthenol | 0.50 |
| Extract of Swertia Herb (Swertiae Herba) | 1.00 |
| Extract of Ginseng Root (Ginseng Radix) | 0.50 |
| Ethanol | 21.00 |
| EDTA-2Na | 0.03 |
| Diisopropanolamine (neutralizing agent) | 0.18 |
| 2% aqueous solution of carboxyvinyl polymer | 9.00 |
| Perfume (including 1-menthol) | 0.05 |
| Preservative | 0.10 |
| Glycinebetaine (humectant) | 1.00 |
| Ion-exchange water (purified water) | balance |

66.7 parts by weight of the above prepared hair growth promoter composition with a viscosity of 7,000 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25°C.

**Example 7**

The following components were mixed to prepare a hair growth promoter composition:

|  | Wt.% |
| --- | --- |
| D-panthenol | 0.50 |
| Extract of Swertia Herb (Swertiae Herba) | 1.00 |
| Extract of Ginseng Root (Ginseng Radix) | 0.50 |
| Ethanol | 21.00 |
| EDTA-2Na | 0.03 |
| Diisopropanolamine (neutralizing agent) | 0.19 |
| 2% aqueous solution of carboxyvinyl polymer | 9.50 |
| Perfume (including 1-menthol) | 0.05 |
| Preservative | 0.10 |
| Glycinebetaine (humectant) | 1.00 |
| Ion-exchange water (purified water) | balance |

66.7 parts by weight of the above prepared hair growth promoter composition with a viscosity of 8,000 cp were mixed with 33.3 parts by weight of dimethyl ether, so that a hair growth promoting aerosol formulation according to the present invention was obtained.

The thus obtained hair growth promoting aerosol formulation was filled into an aerosol container having a nozzle with a diameter of 0.7 mm, so that an aerosol product of the hair growth promoter was prepared. In this case, the internal pressure was 4.3 kg/cm$^2$ at 25°C.

Using the above prepared aerosol products of the hair growth promoters, the following experiments were conducted to evaluate the properties.

**[Experiment 1]**

(Evaluation of the hair setting effect in terms of the curl retention)

A hair sample was prepared by making up a bundle of about 2 g of straight hairs with a length of 23 cm. The hair was fastened with a clip weighing about 10 g at the lower end of the bundle to prevent it from scattering when the spray of the hair growth promoter was applied thereto. The hair sample was set to a motor which was operated at 30 rpm. Each of the aerosol products of the hair growth promoters according to the present invention obtained in Examples 1, 4 and 5, a commercially available hair spray A, and a commercially available hair growth promoter B was sprayed onto the hair sample for 10 seconds at a distance of about 15 cm from the hair sample while rotating the hair sample. Immediately after the completion of spraying, the droplets of the agent attached to the hairs were levelled with a finger and the hair sample was wound around a 1-cm diameter rod. Then, the hair sample was dried at 55°C for one hour, followed by cooling in a desiccator. Thereafter, the rod was removed from the hair sample under the circumstances of 30°C and 80%RH, and the curling hair sample was perpendicularly hung and the length of the curling hair sample was measured every one hour.

The curl retention ratio was obtained in accordance with the following formula:

$$\text{Curl retention ratio (\%)} = \frac{L - L_t}{L - L_0} \times 100$$

wherein L is a length of the hair sample before subjected to curling, that is, 23 cm; $L_0$ is a length of the hair sample immediately after the removal of the rod; and $L_t$ is a length of the hair sample obtained by measuring at arbitrary time.

The results are shown in Figure 1. As can be seen from the graph in Figure 1, it is confirmed that the hair setting effect of the aerosol products of the hair growth promoters according to the present invention is almost equal to that of the commercially available hair spray A, and superior to that of the commercially available hair growth promoter B.

By the addition of a component having a hair setting effect, such as a polyvinyl pyrrolidone derivative, the desired hair setting effect can be obtained and adjusted if necessary when the hair growth promoting aerosol formulation according to the present invention is employed.

**[Experiment 2]**

(Evaluation of dripping of the agent in terms of the adhesion ratio)

A 20 cm x 20 cm glass plate was perpendicularly hung, and each of the aerosol products of the hair growth promoters according to the present invention obtained in Examples 1, 3, 5, 6 and 7, and a commercially available hair growth promoter B of aerosol type was sprayed onto the glass plate for about 3 seconds at a distance of about 15 cm from the glass plate. The glass plate was allowed to stand for 30 seconds as it was. The weight of the glass plate was measured before and after the spraying of the agent, and the weight of each aerosol product of the hair growth promoter was also measured before and after the spraying of the agent. Then, the adhesion ratio of the agent was obtained in accordance with the following formula:

$$\text{Adhesion ratio of agent (\%)} = \frac{G_s - G_0}{E_s - E_0} \times 100$$

wherein $G_s$ is a weight of the glass plate after spraying of the agent; $G_0$ is a weight of the glass plate before spraying of the agent; $E_s$ is a weight of the aerosol product after spraying of the agent; $G_0$ is a weight of the aerosol product before spraying of the agent.

The results are given in the following Table 1:

Table 1

|  | Adhesion Ratio (%) |
|---|---|
| Aerosol product obtained in Example 1 | 60.1 |
| Aerosol product obtained in Example 3 | 58.8 |
| Aerosol product obtained in Example 5 | 59.0 |
| Aerosol product obtained in Example 6 | 64.8 |
| Aerosol product obtained in Example 7 | 60.0 |
| Commercially available aerosol product of hair growth promoter B | 4.7 |

As is apparent from the results in Table 1, it is confirmed that the commercially available aerosol product of hair growth promoter B dripped off the glass plate and little amount of the effective components remained thereon. On the other hand, the adhesion ratio of each hair growth promoting aerosol formulation according to the present invention is high, which proves that the dripping can be prevented.

**[Experiment 3]**

(Organoleptic examination - Practical test)

Each of the aerosol products of the hair growth promoters according to the present invention obtained in Examples 1 to 7 was practically applied to the hairs of ten panelists. The questions whether the hair growth promoter directly reached the scalp or not, whether the hair growth promoter dripped from the scalp or hairs after application thereto or not, and whether the hair setting effect was sufficient or not were put to the panelists.

The results of the above questionnairing are shown in Table 2. The numerals shown in Table 2 indicate the number of panelists.

Table 2

|  |  | Example No. | | | | | | |
|---|---|---|---|---|---|---|---|---|
|  |  | 1 | 2 | 3 | 4 | 5 | 6 | 7 |
| Did the agent directly reach the scalp? | Yes | 10 | 10 | 10 | 10 | 10 | 10 | 10 |
|  | No | 0 | 0 | 0 | 0 | 0 | 0 | 0 |
| Did the agent prevent dripping from the scalp and hairs? | Yes | 10 | 10 | 10 | 10 | 10 | 10 | 10 |
|  | No | 0 | 0 | 0 | 0 | 0 | 0 | 0 |
| Was the hair setting effect satisfactory? | Yes | 10 | 10 | 10 | 10 | 10 | 10 | 10 |
|  | No | 0 | 0 | 0 | 0 | 0 | 0 | 0 |

As can be seen from the results in Table 2, it is confirmed that any of the aerosol products of the present invention can directly reach the scalp by jet-spraying, as well as provide sufficient hair setting effects. Japanese Patent Application No. 5-217981 filed on August 9, 1993 is hereby incorporated by reference.

**Claims**

1. A hair growth promoting aerosol formulation comprising:
   a hair growth promoter composition in the form of a gel, comprising as effective components (a) a crude drug extract having an effect of promoting the circulation of blood and (b) a vitamin or a derivative thereof, and (c) a water-soluble polymer by which said hair growth promoter composition is gelatinized; and
   a propellant.

2. The hair growth promoting aerosol formulation as claimed in Claim 1, wherein said crude drug extract is obtained from at least one selected from the group consisting of Japanese Angelica Root (Angelicae Radix), Cnidium Rhizome (Cnidii Rhizoma), Panax Rhizome (Panacis Japonici Rhizoma), Ginseng Root (Ginseng Radix), Swertia Herb (Swertiae Herba), Ginger Rhizome (Zingiberis Rhizoma), Rehmannia Root (Rehmanniae Radix), Aloe (Aloe), Glycyrrhiza Root (Glycyrrhizae Radix), Garlic, and Capsicum (Capsici Fructus).

3. The hair growth promoting aerosol formulation as claimed in Claim 2, wherein said crude drug extract is obtained from at least one selected from the group consisting of Swertia Herb (Swertiae Herba), Ginseng Root (Ginseng Radix), Glycyrrhiza Root (Glycyrrhizae Radix), and Garlic.

4. The hair growth promoting aerosol formulation of any one of Claims 1 - 3, wherein said vitamin or said derivative thereof is at least one compound selected from the group consisting of tocopherol, vitamin A, vitamin B, vitamin C, vitamin D, vitamin F, vitamin H, vitamin K, vitamin P, vitamin U, D-panthenol (pantothenyl alcohol), carnitine, ferulic acid, $\gamma$-oryzanol, lipoic acid, and orotic acid, and derivatives thereof.

5. The hair growth promoting aerosol formulation as claimed in Claim 4, wherein said vitamin or said derivative thereof is at least one compound selected from the group consisting of D-panthenol, tocopherol and tocopheryl acetate.

6. The hair growth promoting aerosol formulation of any one of Claims 1 - 5, wherein said water-soluble polymer is at least one compound selected from the group consisting of carrageenan, furcellaran, locust-bean gum, xanthane gum, hydroxymethyl cellulose, hydroxyethyl cellulose, hydroxypropyl cellulose, hydroxypropylmethyl cellulose, carboxymethyl cellulose, ethyl cellulose, carboxyvinyl polymer, polyvinyl pyrrolidone, and a copolymer of vinyl pyrrolidone and vinyl acetate.

7. The hair growth promoting aerosol formulation as claimed in Claim 6, wherein said water-soluble polymer is carboxyvinyl polymer.

8. The hair growth promoting aerosol formulation of any one of Claims 1 - 7, wherein the amount of said crude drug extract is in the range of 1.5 to 30 wt.% of the total weight of said hair growth promoter composition.

9. The hair growth promoting aerosol formulation of any one of Claims 1 - 8, wherein the amount of said vitamin or said derivative thereof is in the range of 0.5 to 10 wt.% of the total weight of said hair growth promoter composition.

10. The hair growth promoting aerosol formulation of any one of Claims 1 - 9, wherein said water-soluble polymer is in such an amount that the viscosity of said hair growth promoter composition is in the range of 5,000 to 100,000 cp.

11. The hair growth promoting aerosol formulation as claimed in Claim 10, wherein said water-soluble polymer is in such an amount that the viscosity of said hair growth promoter composition is in the range of 5,000 to 30,000.

12. The hair growth promoting aerosol formulation as claimed in Claim 11, wherein said water-soluble polymer is in such an amount that the viscosity of said hair growth promoter composition is in the range of 8,500 to 30,000 cp.

13. The hair growth promoting aerosol formulation of any one of Claims 1 - 12, wherein said propellant comprises dimethyl ether.

14. The hair growth promoting aerosol formulation as claimed in Claim 13, wherein said propellant comprises dimethyl ether, and at least one gas selected from the group consisting of liquefied natural gas, carbon dioxide gas and chlorofluorocarbon.

15. The hair growth promoting aerosol formulation of any one of Claims 1 - 14, wherein the amount ratio by weight of said hair growth promoter composition to said propellant is in the range of 9:1 to 1:9.

16. The hair growth promoting aerosol formulation of any one of Claims 1 - 15, wherein said crude drug extract is obtained from at least one selected from the group consisting of Swertia Herb (Swertiae Herba), Ginseng Root (Ginseng Radix), Glycyrrhiza Root (Glycyrrhizae Radix), and Garlic, said vitamin or said derivative thereof is at least one compound selected from the group consisting of D-panthenol, tocopherol and tocopheryl acetate, said water-soluble polymer is carboxyvinyl polymer, and said propellant comprises dimethyl ether.

17. An aerosol product of a hair growth promoter prepared by filling a hair growth promoting aerosol formulation comprising a hair growth promoter composition in the form of a gel, comprising as effective components (a) a crude drug extract having an effect of promoting the circulation of blood and (b) a vitamin or a derivative thereof, and (c) a water-soluble polymer by which said hair growth promoter composition is gelatinized, and a propellant into an aerosol container provided with a nozzle having a diameter of 1 mm or less.

18. The aerosol product of said hair growth promoter as claimed in Claim 17, wherein said nozzle of said aerosol container has a diameter of 0.4 to 0.8 mm.

# FIGURE